# EUROPEAN PATENT APPLICATION

(11) **EP 4 843 570 A1**
(43) Date of publication of application: **25.11.2026**
(21) Application number: 24918209.8
(22) Date of filing: 18.11.2024
(51) Int. Cl.: A61K 31/315, A61K 35/15, A61K 9/08, A61P 29/00, A61P 37/06, A61P 37/08

(54) **USE OF AMINO ACID CHELATED ZINC IN IMMUNOMODULATION AND TREATING AUTOIMMUNE DISEASES**

(30) Priority: 18.01.2024 US 202463622205 P
(71) Applicant: FAAC BIO INC., New Taipei City (TW)
(72) Inventor: HSU, Hsin-ching, New Taipei City Taiwan (TW); HSU, Li-chuan, New Taipei City Taiwan (TW); LIN, Wei-yu, New Taipei City Taiwan (TW); FU, Hao-chuan, New Taipei City Taiwan (TW)
(74) Representative: Herrero & Asociados, S.L.
(86) International application number: PCT/CN2024/132566
(87) International publication number: WO 2025/152600

(57) **Abstract**

Use of amino acid-chelated zinc in the manufacture of a composition for immunomodulating and /or inhibiting dendritic cell maturation is provided, the use is particularly for the manufacture of a pharmaceutical composition for treating allergic diseases, treating autoimmune diseases, and/or preventing organ transplant rejection; a combination for preparing tolerogenic dendritic cells is also provided, the combination comprises (1) amino acid-chelated zinc and (2) a dendritic cell culture solution.

## Description

### FIELD OF THE INVENTION

The present invention relates to the use of an amino acid-chelated zinc, particularly the use of an amino acid-chelated zinc on immune regulation in a subject, especially to provide a subject in need (e.g., patients with autoimmune disease, organ transplant recipients) with the benefit(s) of inhibiting immune response.

### BACKGROUND OF THE INVENTION

According to statistics made by the Autoimmune Association in the USA, more than 100 autoimmune diseases have been discovered and more than fifty million people in the USA are suffering from autoimmune diseases. Moreover, the number of patients suffering from organ failure is increasing year by year due to the gradual aging of social structure and changes in modern lifestyle. Clinically, organ transplant is the most effective method for treating an end-stage organ failure. It is expected that the global organ transplantation market will reach US$61.5 billion by 2027 and increase at a compound annual growth rate of 9.9% over the forecast period. To prevent the recipient's immune system from rejecting the transplanted organ, the recipient usually needs to take an immunosuppressant over a long period of time. Therefore, the industry has been committed to developing medicaments that are effective in regulating immunity, particularly inhibiting undesired immune response(s).

Dendritic cell (DC) is a professional antigen-presenting cell (APC), which serves as a bridge between the innate immune response and the adaptive immune system. When an exogenous antigen is recognized by the immune system, DC will mature, leading to an increase in the expressions of costimulatory factors such as CD80, CD86 and CD40, and major histocompatibility complex class II (MHC II) on the surface of DC, and secret proinflammatory cytokine to activate downstream T cells, thereby producing cytotoxic effects and clearing exogenous antigens.

In recent years, it was found from studies that DC with low expressions of costimulatory factors and MHC II (i.e., semi-mature DC) has the ability to induce immune tolerance, thereby, allowing T cell anergy, activating regulatory T cell (Treg cell), and secreting anti-inflammatory factors. Such semi-mature DC is also known as tolerogenic DC. Because of the anti-inflammatory and immunosuppressive functions of tolerogenic DC, tolerogenic DC is also considered as a potential cell therapy for treating allergic diseases, treating autoimmune diseases (such as type1 diabetes, multiple sclerosis, rheumatoid arthritis, etc.) or preventing an organ transplant rejection.

### SUMMARY OF THE INVENTION

Inventors of the present invention found that amino acid-chelated zinc has an excellent effect on inhibiting dendritic cell (DC) maturation. As compared to the known active ingredients capable of inhibiting dendritic cell maturation (e.g., Zinc sulfate), the amino acid-chelated zinc can provide a desired inhibitory effect at a relatively low concentration of zinc. Inventors of the present invention also found that the administration of amino acid-chelated zinc in a subject with an autoimmune disease can effectively alleviate the subject's symptom(s) resulted from the autoimmune disease.

Therefore, an objective of the present invention is to provide the use of an amino acid-chelated zinc in the manufacture of a composition, wherein the composition is used for immune regulation. In some embodiments, the composition is a pharmaceutical composition or a food composition. In some embodiments, the pharmaceutical composition is for treating an allergic disease, treating an autoimmune disease, and/or preventing an organ transplant rejection.

In some embodiments of the use of the present invention, the composition is an aqueous composition with a pH less than 7.

Another objective of the present invention is to provide the use of an amino-chelated zinc in the manufacture of a composition, wherein the composition is for inhibiting dendritic cell maturation.

Still another objective of the present invention is to provide a combination, comprising:
(1) an amino acid-chelated zinc; and
(2) a dendritic cell medium.

In some embodiments of the combination in accordance with the present invention, the combination further comprises a dendritic cell.

In some embodiments of the combination in accordance with the present invention, based on the amount of the dendritic cell medium, the amino acid-chelated zinc has an amount of zinc ranging from 1 mg to 25 mg per mL of the dendritic cell medium.

Still another objective of the present invention is to provide a method for regulating immunity and/or inhibiting dendritic cell maturation, which comprises administering an effective amount of the composition as mentioned above to a subject in need.

In some embodiments of the method for immune regulation in accordance with the present invention, the method is for treating an allergic disease, treating an autoimmune disease, and/or preventing an organ transplant rejection.

Still another objective of the present invention is to provide a method for preparing a tolerogenic dendritic cell, comprising contacting the tolerogenic dendritic cell with an effective amount of the amino acid-chelated zinc. In some embodiments of the method, the contact is performed by culturing the dendritic cell in a dendritic cell medium containing the amino acid-chelated zinc.

In some embodiments of the use, combination and method in accordance with the present invention, the amino acid-chelated zinc has a molar ratio of zinc and amino acid ranging from 1: 1 to 1: 10.

In some embodiments of the use, combination and method in accordance with the present invention, the amino acid-chelated zinc is Glycine-chelated zinc, Alanine-chelated zinc, Valine-chelated zinc, Leucine-chelated zinc, and/or Isoleucine-chelated zinc.

To render the above objectives, technical features and advantages of the present invention more apparent, the present invention will be described in detail with reference to some embodiments hereinafter.

### DESCRIPTION OF THE DRAWINGS

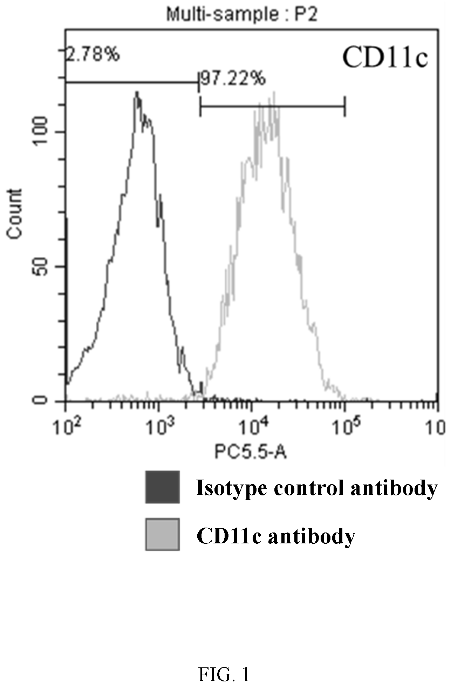

Fig. 1 is a graphical representation of flow cytometry results for analyzing the expression level of CD11c in the isolated mouse bone marrow dendritic cells (BMDC), wherein 97.22% of the cells expressed CD11c and only 2.78% of the cells didn't express CD11c. This result indicates that the purity of BMDC is quite high.

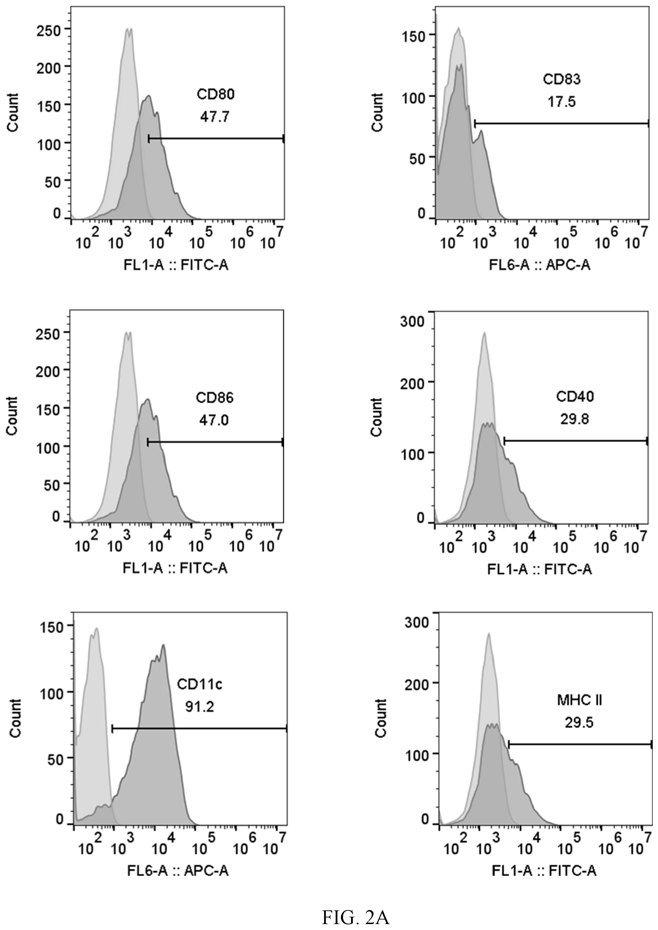

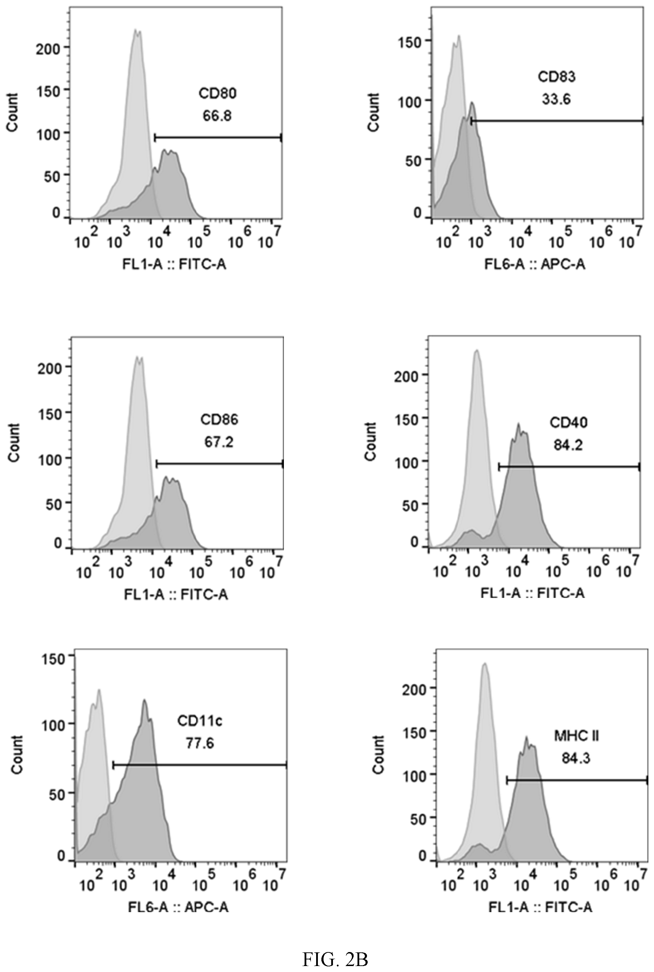

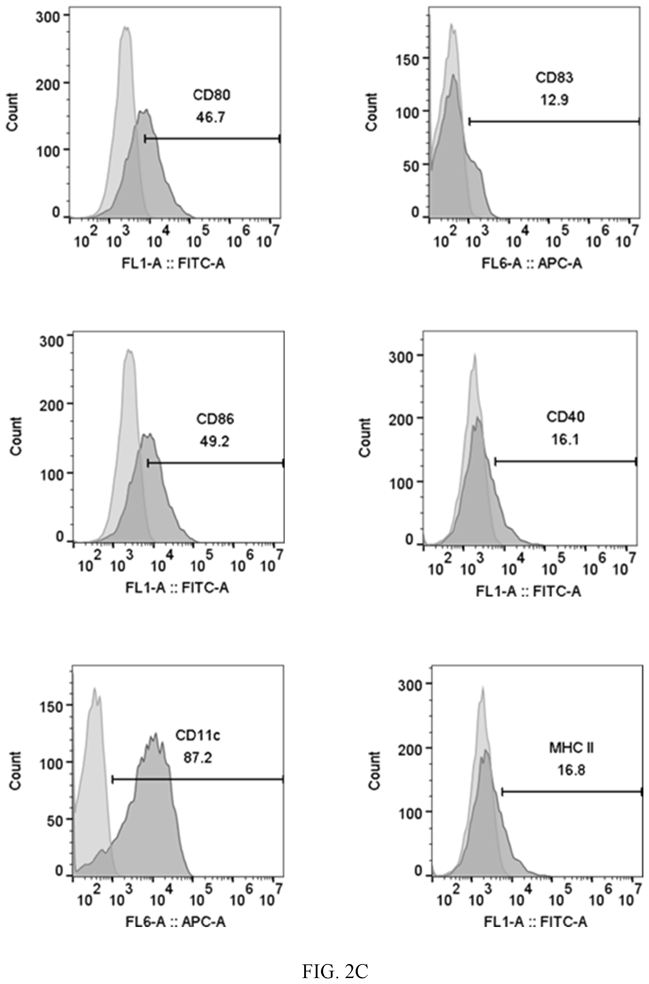

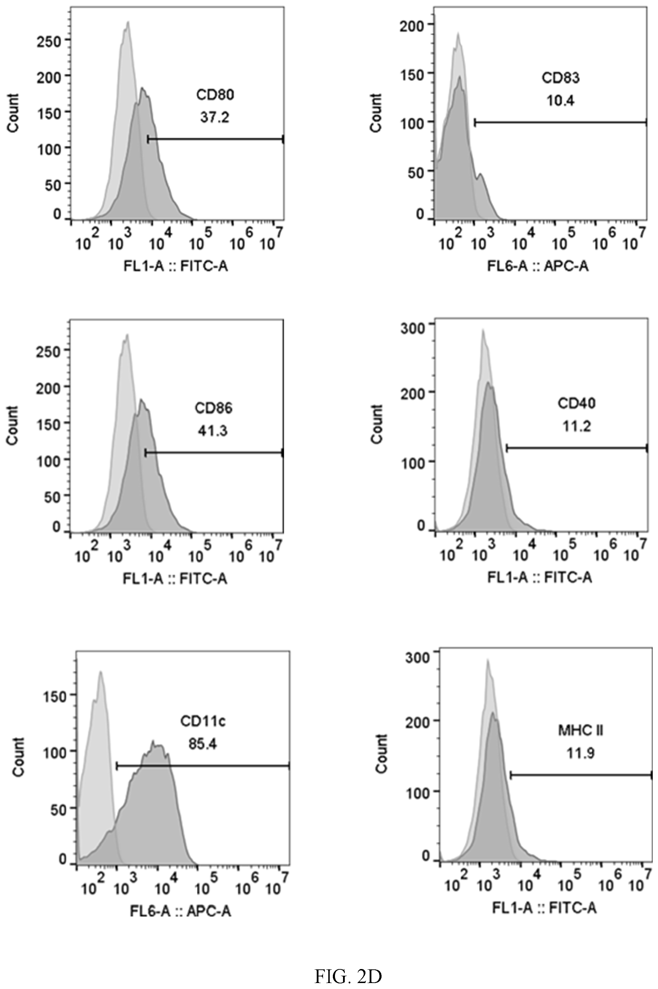

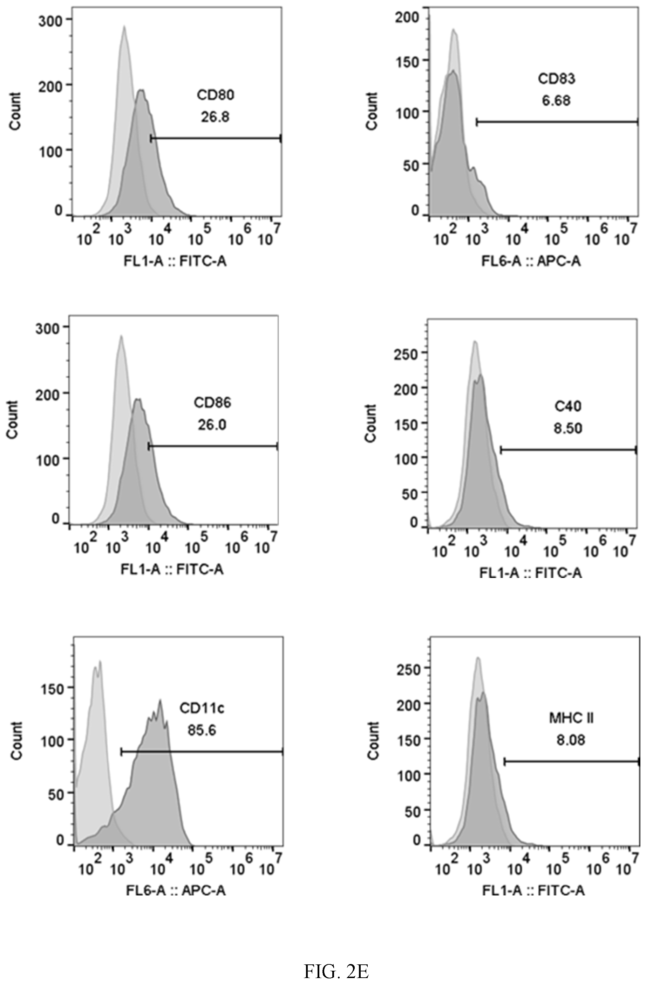

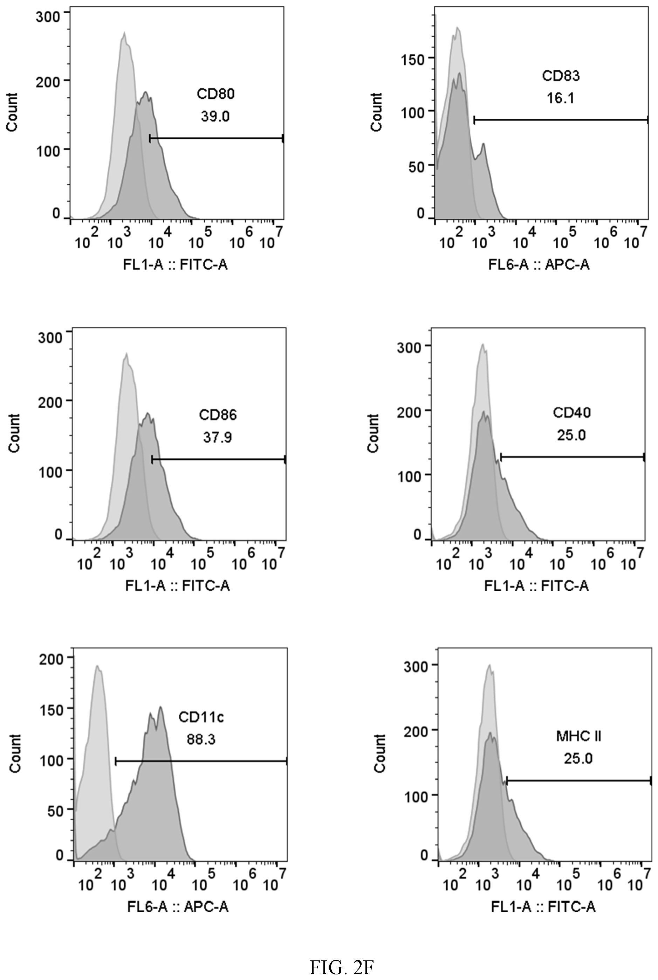

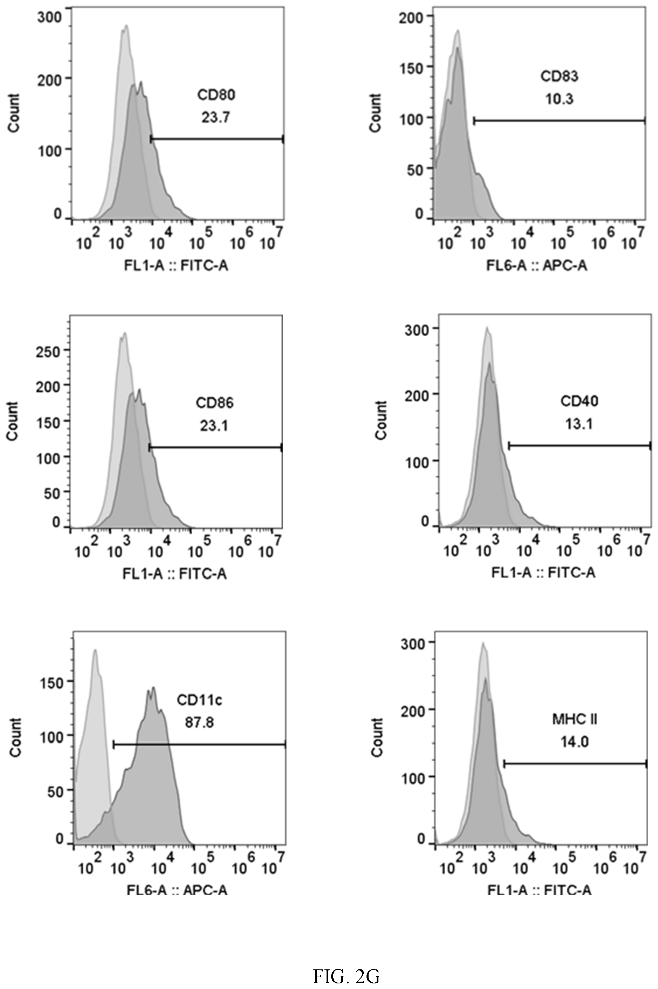

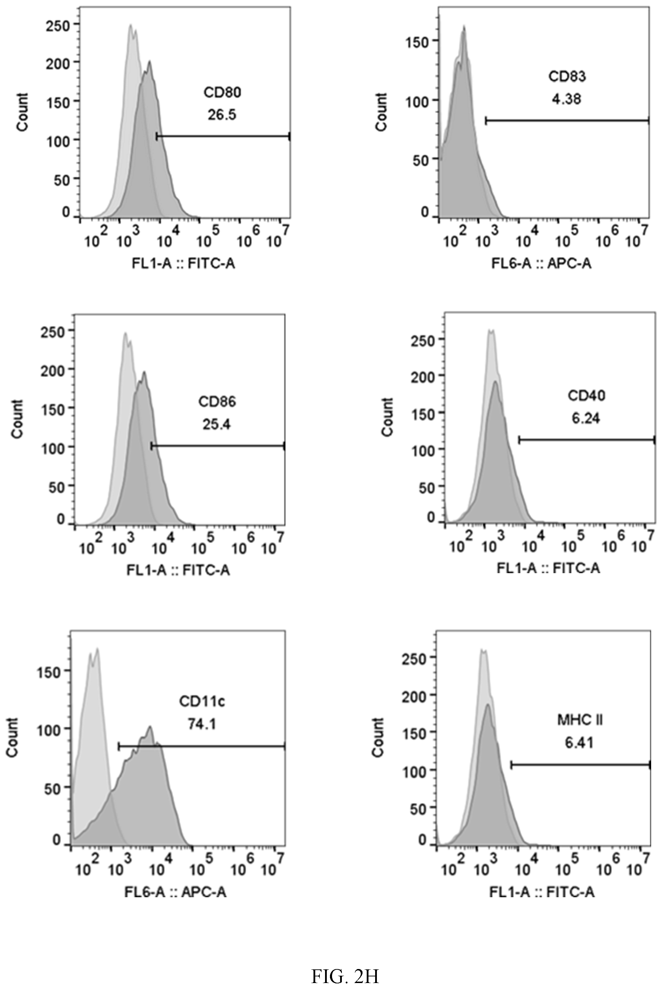

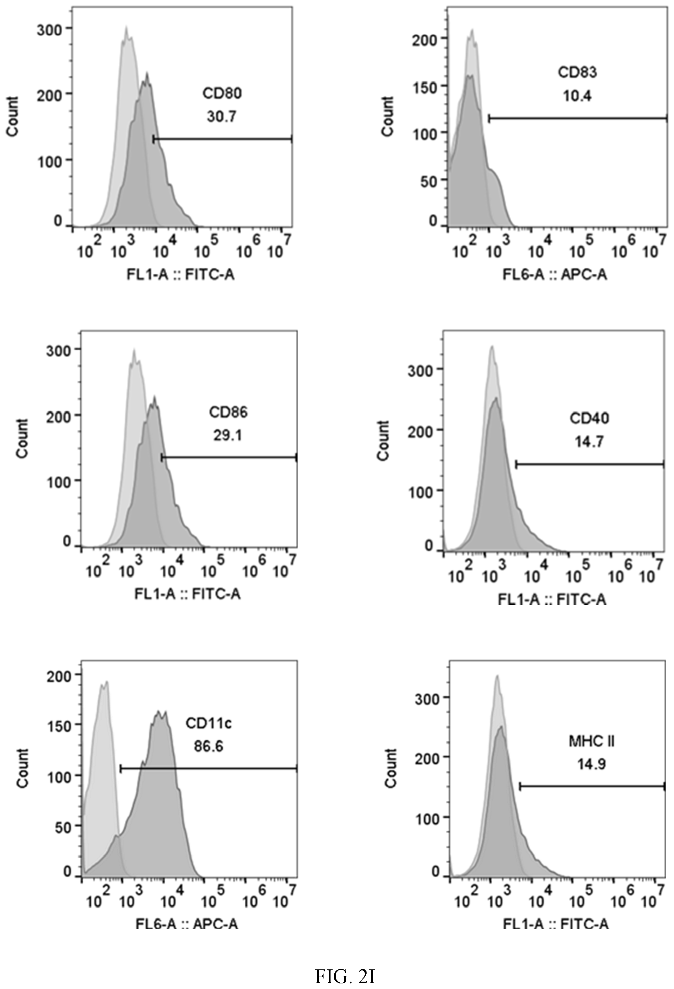

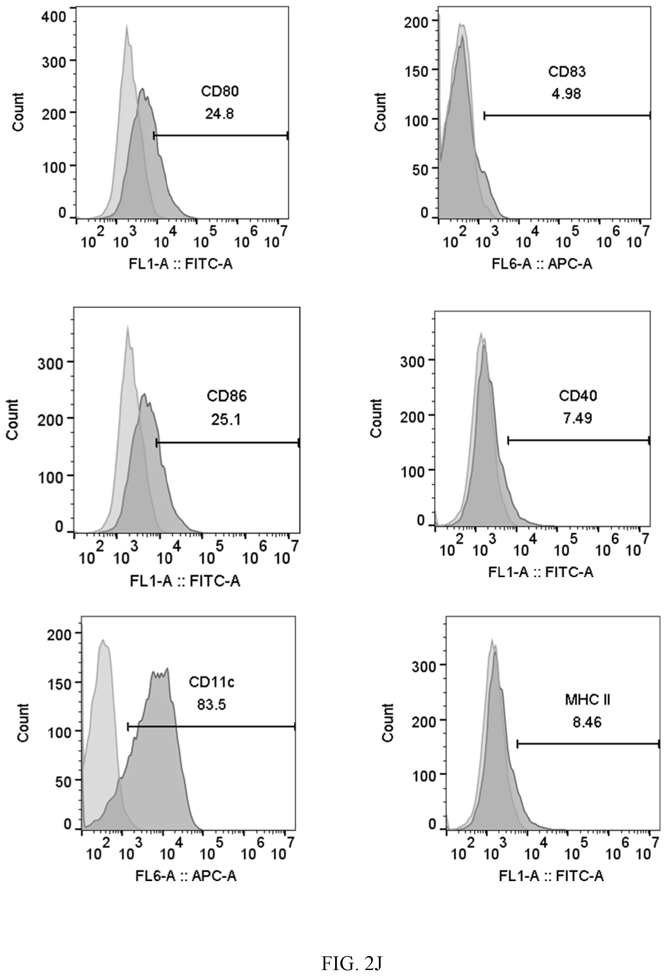

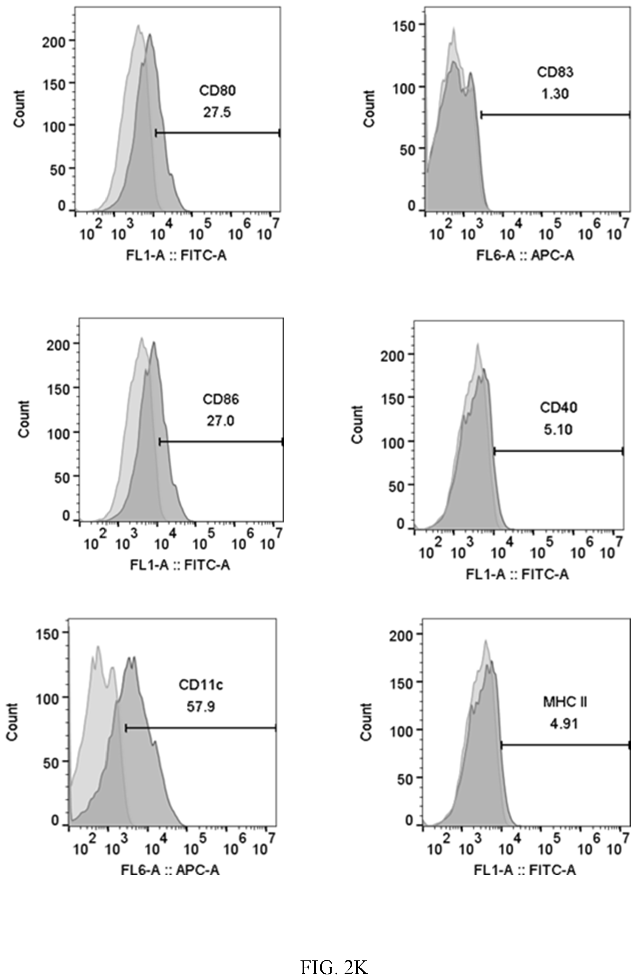

Figs. 2A to 2K are graphical representations of flow cytometry results for analyzing the expression levels of CD80, CD83, CD86, CD40, CD11c and MHC II on the cell surfaces of BMDC with different treatments, wherein Fig. 2A shows the result of untreated BMDC (i.e., immature DC), Fig. 2B shows the result of activated BMDC (i.e., mature DC), Fig. 2C shows the result of BMDC treated with zinc sulfate at a zinc concentration of 3.27 mg/mL, Fig. 2D shows the result of BMDC treated with zinc sulfate at a zinc concentration of 6.54 mg/mL, Fig. 2E shows the result of BMDC treated with zinc sulfate at a zinc concentration of 13.08 mg/mL, Fig. 2F shows the result of BMDC treated with a solution of glycine-chelated zinc (CMC-Zn) at a zinc concentration of 3.27 mg/mL, Fig. 2G shows the result of BMDC treated with CMC-Zn at a zinc concentration of 6.54 mg/mL, Fig. 2H shows the result of BMDC treated with CMC-Zn at a zinc concentration of 13.08 mg/mL, Fig. 2I shows the result of BMDC treated with modified CMC-Zn at a zinc concentration of 3.27 mg/mL, Fig. 2J shows the result of BMDC treated with modified CMC-Zn at a zinc concentration of 6.54 mg/mL and Fig. 2K shows the result of BMDC treated with modified CMC-Zn at a zinc concentration of 13.08 mg/mL.

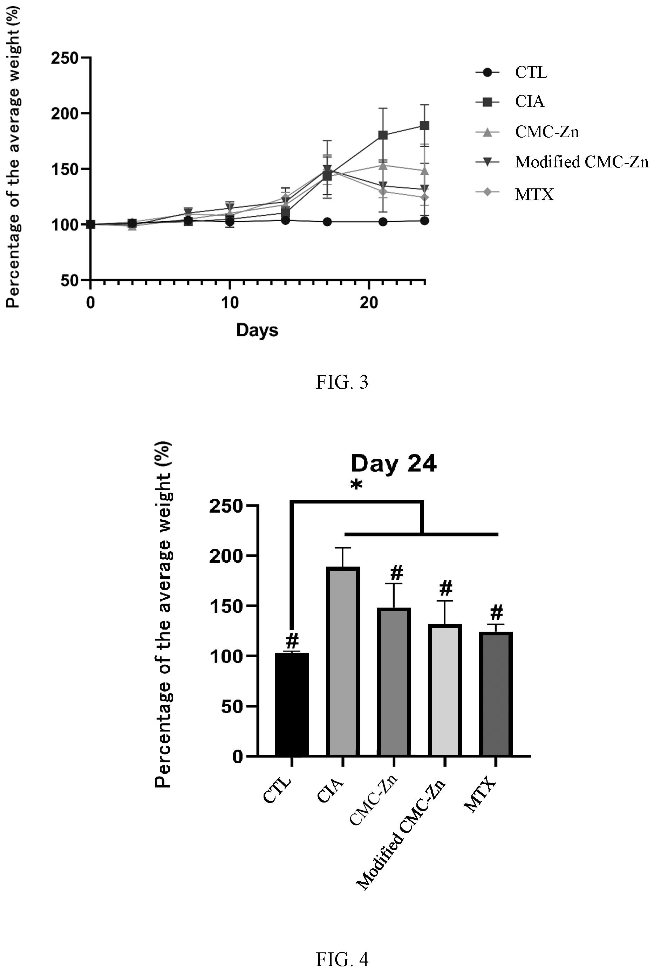

Fig. 3 is a line chart depicting the average weight percentage of both paws of rats as a function of time, wherein the control group includes the untreated healthy rats, the CIA group includes rats with collagen-induced arthritis, the MTX group includes arthritis rats administered with MTX from Day 17, the CMC-Zn group includes arthritis rats administered with the CMC-Zn from Day 17, and the modified CMC-Zn group includes arthritis rats administered with the modified CMC-Zn from Day 17.
Fig. 4 is a bar chart showing the average weight percentage of both paws of rats on Day 24, wherein the rats in each group were treated in the same way as those of Fig.3. "*" represents that the result has a significant difference to that of the control group (p value<0.05), and "#" represents that the result has a significant difference to that of the CIA group (p value<0.05).

### DESCRIPTION OF THE PREFERRED EMBODIMENTS

Hereinafter, some embodiments in accordance with the present invention will be described in detail. However, without deviating from the spirit of the present invention, the present invention may be embodied in various embodiments and should not be limited to the embodiments described in the specification.

Unless otherwise explained in the text, the terms "a," "an," "the," or the like recited in the specification (especially in the claims) should include both the singular and the plural forms; the term "subject" recited herein refers to a human or a non-human mammal. Examples of the non-human mammal include, but are not limited to, bovine, horse, sheep, goat, pig, donkey, mule, dog, cat, rabbit, rodent (such as hamster, guinea pig etc.), ape, monkey, and orangutan.

The numerical ranges (e.g., 5 to 100) used herein should be construed as including all of the rational numbers within the ranges and ranges consisting of any rational numbers within the ranges. Therefore, the numerical ranges used herein should include all the possible combinations of numerical values between the listed minimum and maximum values.

As mentioned above, inventors of the present invention found that the amino acid-chelated zinc has excellent effect on inhibiting DC maturation, and the administration of amino acid-chelated zinc to a subject with an autoimmune disease can alleviate the subject's symptom(s) resulted from the autoimmune disease. Therefore, the present invention relates to the use of the amino acid-chelated zinc on regulating immunity and/or inhibiting DC maturation.

### Amino acid-chelated zinc

The amino acid-chelated zinc used in the present invention can be a commercially available product or a product of a synthesis method. Methods suitable for synthesizing the amino acid-chelated zinc are well known to those skilled in the art. For example, the chelation of amino acid and zinc can be performed by operations such as physically mixing and/or heating to obtain the amino acid-chelated zinc. The molar ratio of zinc to amino acid in the amino acid-chelated zinc used in the present invention can range from 1: 1 to 1: 10, such as 1: 1, 1: 2, 1: 3, 1: 4, 1: 5, 1: 6, 1: 7, 1: 8, 1: 9 or 1: 10 (zinc: amino acids). In some embodiments, the molar ratio of zinc to amino acid is 1: 1 to 1: 4.

There is no specific limitation on the amino acid used in the preparation of the amino acid-chelated zinc for the present invention as long as the desired effect(s) of the present invention (i.e. regulating immunity and/or inhibiting DC maturation) is not affected. Examples that can be used to prepare the amino acid-chelated zinc include glycine, alanine, valine, leucine, and/or isoleucine, but are not limited thereby. The amino acid-chelated zinc used in the present invention can be prepared from a zinc-containing inorganic compound. Examples of the zinc-containing inorganic compound include, but are not limited to, zinc sulfate, zinc carbonate, zinc oxide, zinc chloride and the like.

In some embodiments, the amino acid-chelated zinc used in the present invention can be prepared by sequentially conducting the steps of mixing an amino acid and a zinc source, refrigerating and heating. Preferably, the refrigerating step comprises placing the mixture obtained from the mixing step at 0°C to 4°C for at least 8 hours, such as placing at 0°C to 4°C for 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 36 hours, or 48 hours, or within a range between any two of the values described herein. Preferably, the heating step comprises heating the refrigerated mixture at 50°C to 100°C for 6 hours to 24 hours, such as heating at 50°C, 52.5°C, 55°C, 57.5°C, 60°C, 62.5°C, 65°C, 67.5°C, 70°C, 72.5°C, 75°C, 77.5°C, 80°C, 82.5°C, 85°C, 87.5°C, 90°C, 92.5°C, 95°C, 97.5°C or 100°C for 6 hours, 6.5 hours, 7 hours, 7.5 hours, 8 hours, 8.5 hours, 9 hours, 9.5 hours, 10 hours, 10.5 hours, 11 hours, 11.5 hours, 12 hours, 12.5 hours, 13 hours, 13.5 hours, 14 hours, 14.5 hours, 15 hours, 15.5 hours, 16 hours, 16.5 hours, 17 hours, 17.5 hours, 18 hours, 18.5 hours, 19 hours, 19.5 hours, 20 hours, 20.5 hours, 21 hours, 21.5 hours, 22 hours, 22.5 hours, 23 hours, 23.5 hours or 24 hours. The suitable heating temperature and heating time can also be within a range between any two of the values described herein. In some embodiments, a drying step (e.g., thermal drying or lyophilization) can be further conducted after the heating step to provide the amino acid-chelated zinc in powder form.

### Composition for regulating immunity/inhibiting DC maturation

As mentioned above, the amino acid-chelated zinc is effective in regulating immunity and/or inhibiting DC maturation, and thus can be used to provide a composition for regulating immunity and/or inhibiting DC maturation.

Therefore, in an aspect of the present invention, a composition for regulating immunity and/or inhibiting DC maturation is provided, wherein the composition comprises an effective amount of the amino acid-chelated zinc. In some embodiments of the composition provided in accordance with the present invention, amino acid-chelated zinc can be mixed with water (e.g., sterile water, pure water) or an aqueous solution to form an aqueous composition. Examples of the aqueous composition include, but are not limited to, saline, phosphate buffered saline (PBS) and acidic aqueous solution (e.g., sulfate acid aqueous solution, hydrochloric acid aqueous solution, phosphoric acid aqueous solution, acetic acid aqueous solution, citric acid aqueous solution). Preferably, the acidic aqueous solution is an acidic aqueous solution in compliance with food grade requirements, such as aqueous solution of sulfuric acid of food grade, aqueous solution of hydrochloric acid of food grade, aqueous solution of phosphoric acid of food grade, and the like. In some embodiments, the amino acid-chelated zinc is mixed with an acidic aqueous solution to provide an aqueous composition with a pH less than 7, such as pH 1, pH 1.5, pH 2, pH 2.5, pH 3, pH 3.5, pH 4, pH 4.5, pH 5, pH 5.5, pH 6, pH 6.5 or 6.9 or within a range between any two of the values described herein.

Optionally, the pH can be adjusted depending on the requirement(s) for practical use of the composition (e.g., subjects to be administered, administration routes etc.) For example, when the composition is administered to a human or a mouse/rat via oral administration, the composition can be an aqueous composition with a pH of 2; when the composition is administered to a mature pig, the composition can be an aqueous composition with a pH of 3.5; and, when the composition is administered to a sucking pig, the composition can be an aqueous composition with a pH of 4.5.

The amount of amino acid-chelated zinc in the composition provided in accordance with the present invention can be determined based on the daily zinc intake required by the subject(s) to be administered. In general, the daily zinc intake for a healthy adult female is 12 mg, the daily zinc intake for a healthy adult male is 15 mg, and the upper limit of the daily zinc intake is 35 mg. In addition, the amount of amino acid-chelated zinc in the composition can also be optionally adjusted depending on desired administration frequencies, such as once a day, multiple times a day, or once every few days.

In some embodiments of the composition provided in accordance with the present invention, the composition is a pharmaceutical composition, and the pharmaceutical composition can be used for treating an allergic disease, treating an autoimmune disease and/or preventing an organ transplant rejection, or can be used for alleviating discomfort resulted from the aforementioned disease(s). Examples of the allergic disease include, but are not limited to, allergic rhinitis, allergic conjunctivitis, atopic dermatitis, urticaria, allergic gastroenteritis and asthma. Examples of the autoimmune disease include, but are not limited to, rheumatoid arthritis, type 1 diabetes, multiple sclerosis, myasthenia gravis, systemic lupus erythematosus and ankylosing spondylitis.

The pharmaceutical composition provided in accordance with the present invention can be administered systemically or topically, and can be delivered by various drug delivery system (DDSs). Suitable drug delivery systems include, but are not limited to, an oral drug delivery system and an injectable drug delivery system. For example, the pharmaceutical composition can be administered to a subject in need by oral administration, intravenous injection (including drip infusion and bolus injection), intramuscular injection, subcutaneous injection, arterial injection or intraperitoneal injections, but is not limited thereby. In addition, the pharmaceutical composition provided in accordance with the present invention can be delivered in the form of liposome, microcapsule, nanoparticle, and the like to enhance bioavailability, control drug release speed, target the lesion precisely and reduce side effects.

The pharmaceutical composition provided in accordance with the present invention can be in any suitable form without special limitation, and can be presented in an appropriate dosage form depending on the desired purpose(s). For example, the pharmaceutical composition can be available as a suspension, an emulsion, a solution (such as syrup, elixirs, tincture, etc.), coated tablets, tablets, granules, powders, capsules, pellets, pills and an injection, but is not limited thereby. Depending on the form and purpose(s), a pharmaceutically acceptable excipient can be chosen to provide the pharmaceutical composition, wherein, the excipients are well known by a person skilled in the art of pharmaceutical manufacturing, and include, but are not limited to, a diluent, a surfactant, a flow agent, a disintegrating agent, an adhesive, a buffer, a colorant, a flavoring agent, an antioxidant, a preservative and a film forming agent.

In some embodiments of the composition provided in accordance with the present invention, the composition can be a food composition, and the food composition can be used for immune regulation. The food composition can be a beverage, a solid food or a semi-solid food, and can be provided in a form of a health food, a daily supplement, a functional food, a nutritional supplement or a special nutrition food. In some embodiments, the food composition is a health food.

Depending on the form and purpose(s), the food composition in accordance with the present invention can comprise any suitable food additive(s). Examples of the food additive include, but are not limited to, a preservative, a bactericide, an antioxidant, a bleaching agent, a color fasting agent, a swelling agent, a nutrition additive, a colorant, a flavoring agent (e.g., a sweetener), a pasting agent, a binding agent, chemicals for food industry, an emulsifier, and agents for food quality improvement, fermentation and food processing.

### Method for regulating immunity/inhibiting DC maturation

In another aspect of the present invention, a method for regulating immunity and/or inhibiting DC maturation is provided, wherein the method comprises administering an effective amount of the composition described above to a subject in need.

In the method for regulating immunity and/or inhibiting DC maturation in accordance with the present invention, wherein the subject in need refers to a subject with immune imbalance (especially abnormal immune activation, e.g., patients with autoimmune diseases), or a subject who has undergone an organ transplant.

### Combination for preparing a tolerogenic DC

In still another aspect of the present invention, provided is a combination, which comprises: (1) an amino acid-chelated zinc; and (2) a dendritic cell medium, wherein, the combination can be used for preparing a tolerogenic DC, and the amino acid-chelated zinc is defined as above.

In the combination provided in accordance with the present invention, there is no specific limitation on the dendritic cell medium as long as it can provide the nutrient(s) required for cultivating a dendritic cell. In some embodiments, the dendritic cell medium is RPMI-1640 medium comprising interleukin-4 (IL-4), granulocyte-macrophage colony-stimulating factor (GM-CSF) and fetal bovine serum (FBS).

In some embodiments of the combination provided in accordance with the present invention, the combination further comprises a dendritic cell, and the dendritic cell is preferably an immature dendritic cell.

In some embodiments of the combination provided in accordance with the present invention, based on the amount of the dendritic cell medium, the amino acid-chelated zinc has an amount of zinc ranging from 1 mg to 25 mg per mL of the dendritic cell medium. For example, based on the amount of the dendritic cell medium, the amino acid-chelated zinc has an amount of zinc is 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21.5 mg, 22 mg, 22.5 mg, 23 mg, 23.5 mg, 24 mg, 24.5 mg or 25 mg per mL of the dendritic cell medium, or within a range between any two of the values described herein.

The combination provided in accordance with the present invention can be a kit or a composition. When the combination provided in accordance with the present invention is a kit, the component (1) amino acid-chelated zinc, and the component (2) dendritic cell medium, are usually packaged separately and stored independently in different containers (e.g., a plastic bag, a plastic bottle, a glass bottle, an ampoule), and can be transported or sold either alone or in combination as a set. The kit can further comprise an instruction manual, which provides the procedures and program for the user to mix the components on-site for cell culture, processing and application.

When the component (1) and the component (2) are packaged separately and stored independently in different containers, they can be stored at different environments. For example, the container of the component (1) can be stored at room temperature or a temperature of 0°C to 7°C or -20°C; the container of the component (2) can be stored at a temperature of 0°C to 7°C.

In addition, when the combination provided in accordance with the present invention is a composition, the component of (1), amino acid-chelated zinc, and the component (2), dendritic cell medium, are usually mixed and stored in a same container (e.g., a plastic bag, a plastic bottle, a glass bottle, an ampoule).

When the combination provided in accordance with the present invention further comprises a dendritic cell, the dendritic cell can be stored in a cell storage container known in the art (e.g., a cryotube). In some embodiments, the dendritic cell is mixed with a cryoprotective agent and placed into a cryotube to store at -80°C or in liquid nitrogen. The cryoprotective solution is well known in the art, such as a cell culture medium containing 5% to 10% by volume of Dimethyl sulfoxide (DMSO), or a commercially available cryoprotective solution.

### Method for preparing a tolerogenic DC

In still another aspect of the present invention, a method for preparing a tolerogenic DC is provided, wherein the method comprises contacting a dendritic cell with an effective amount of an amino acid-chelated zinc.

In some embodiments, the contact is performed by culturing the dendritic cell in a dendritic cell medium containing the amino acid-chelated zinc. Furthermore, the amino acid-chelated zinc, dendritic cell and dendritic cell medium are defined as above.

The present invention will be further illustrated in detail with specific examples as follows, wherein the following examples are provided only for illustrating the present invention and the scope of the present invention is not limited thereby. The scope of the present invention will be indicated in the appended claims.

### Examples

The materials and equipment as used in the following examples are listed as below.
(1) Zinc sulfate: purchased from SIGMA Co., product no.: 1282101001.
(2) PBS: purchased from Corning Co., product no.: 21-040-CM.
(3) Mouse: purchased from BioLASCO Taiwan Co., Ltd., strain: C57BL/6.
(4) Lewis rats: purchased from BioLASCO Taiwan Co., Ltd.,7 week-old, female.
(5) RPMI-1640 medium: purchased from Thermo Fisher Co., product no.: 11875093.
(6) Red blood cell lysis buffer (RBC lysis buffer): purchased from SIGMA Co., product no.: R7757.
(7) DC medium: RPIM-1640 medium containing 500 U/mL of IL-4 (purchased from Thermo Fisher Co., product no.: 214-14), 1000 U/mL of GM-CSF (purchased from Thermo Fisher Co., product no.: 315-03) and 10% (v/v) of FBS (purchased from Thermo Fisher Co., product no.: A3160402).
(8) Flow cytometry: Brand CytoFLEX S, Model: Beckman.
(9) Collagen: purchased from Chondrex Co., Immunization Grade Bovine Type II Collagen.
(10) Freund's Incomplete Adjuvant (IFA): purchased from SIGMA Co.
(11) Interferon-γ (IFN-γ): purchased from Thermo Fisher Co., product no.: 315-05.
(12) R848 (resiquimod): purchased from MCE Co., product no.: HY-13740.
(13) Polyinosinic- Polycytidylic acid (poly I:C): purchased from MCE Co., product no.: HY-107202.
(14) Lipopolysaccharide (LPS): purchased from SIGMA Co., product no.: L2630.
(15) Analytical reagents and antibodies for flow cytometry: FcRBlocking Reagent (purchased from MACS Co., product no.: 130-092-575), CD11c antibody (purchased from biolegend Co., product no.: 117309), CD40 antibody (purchased from biolegend Co., product no.:124607), CD80 antibody (purchased from biolegend Co., product no.: 104705), CD83 antibody (purchased from biolegend Co., product no.: 121509), CD86 antibody (purchased from biolegend Co., product no.: 105031), MHC II antibody (purchased from biolegend Co., product no.: 107631), isotype control (purchased from biolegend Co., product no.: 400905, 400411, 400535).
(16) ELISA analysis kit for IL12P70: purchased from Arigo Co., product no.: ARG80201, including a 96-well plate pre-coating IL12P70 antibodies, a IL12P70 standard, a Wash buffer, an Antibody conjugate, a HRP-Streptavidin solution, a TMB reagent and a Stop Solution. Before using, each reagent/solution is placed at room temperature for 20 minutes.
(17) Methotrexate (MTX): purchased from MCE Co., product no.: HY-14519

### [Preparation Example]

### A. Preparation of amino acid-chelated zinc

The amino acid-chelated zinc used in the examples is prepared by the following method: sieving an aqueous solution of zinc sulfuric acid of food grade and a glycine of food grade with a 30 mesh sieve, respectively, and mixing at a molar ratio ranging from 1: 1 to 1: 4; refrigerating the obtained mixture at 0°C to 4°C for 12 hours to 24 hours; and, heating the refrigerated mixture at a temperature of 80°C for 12 hours to obtain a glycine-chelated zinc powder.

For the cell experimentation of Example 1, the glycine-chelated zinc powder was adjusted with the DC medium to form solutions at a zinc concentration of 13.08 mg/mL, 6.54 mg/mL and 3.27 mg/mL, respectively, and for the animal experimentation of Example 2, the glycine-chelated zinc powder was dissolved into 2 mL of PBS to obtain a solution containing 0.075 mg zinc (hereinafter referred to as "CMC-Zn"). In addition, the pH values of the aforesaid CMC-Zn at different concentrations of zinc were adjusted with citric acid to 2, respectively (hereinafter referred to as "modified CMC-Zn").

### Preparation of mouse bone marrow dendritic cells (BMDC)

Mice were euthanized with carbon dioxide and immersed in 75% alcohol. After immersing for 3 minutes, the mice were removed from the alcohol and wiped dry. Then, the mouse limbs were spread and fixed on an operation board. Next, the entire femur and tibia were collected by carefully cutting off the mouse skin and muscle. A 100 micrometer (µm) filter was placed in a 3-centimeter culture dish and 2 mL of RPMI-1640 medium was added. Two ends of the femur were cut, and the bone marrow was collected by drawing RPMI-1640 medium from the culture dish with a 26G syringe and inserting into the bone to repeatedly wash the femur several times until the femur appeared pure white. The bone marrow in the tibia was also washed onto the filter using the same method. Then, the filter was scraped several times by the reverse side of 1 mL micropipette tip to break up the cell clumps in the bone marrow, and washed the filter with the culture medium in the culture dish several times. Then, the culture medium in the culture dish was collected and placed into a centrifuge tube to centrifuge at 1500 rpm for 3 minutes. After centrifuging, the supernatant was removed and 1 mL of RBC lysis buffer was added to re-suspend the red pellet. After standing for 5 minutes, the tube was centrifuged at 1500 rpm for 3 minutes again. After removing the supernatant, a nearly milky-white pellet was obtained. The pellet was re-suspended with the DC medium and the cell number of the suspension thus obtained was counted.

The cell concentration of the suspension was adjusted to 5×10⁵ cells per mL, and 1 mL of the suspension was added into each well of a 24-well plate. The 24-well plate was incubated at 37°C and 5% CO₂. After 2 days, the unattached granulocytes were removed by carefully removing the culture medium, and then, 0.5 mL of DC medium was added into each well. After incubating 3 days, the culture medium in each well was replaced with 0.5 mL of fresh DC medium and continuously incubated for 3 days. The 24-well plate was taken out from the incubator, and the medium in each well was carefully removed. The bottom of each well was gently pipetted with 2 mL of PBS several times. The pipetted PBS was collected into a centrifuge tube to count the number of cells and analyze the expression level of CD11c of the obtained cells using flow cytometry.

As shown in Fig. 1, the expression level of CD11c in the obtained cells is more than 70% as analyzed by flow cytometry, and thus the cells are served as BMDC for subsequent experimentations.

### C. Preparation of rats with rheumatoid arthritis

Lewis rats were bred under the condition with a temperature of 21°C to 25°C, a humidity level of 40% to 70%, and a lighting period of 12 hours (7 a.m. to 7 p.m.). The rats had free access to the feed. The rats were monitored for at least 6 days to ensure their health to prepare a collagen-induced arthritis (CIA) animal model.

An inducing emulsion was prepared by evenly mixing collagen and IFA at a volume ratio of 1: 1 using a homogenizer in a cold water bath. On Day 1, 200 microliters (µL) of the inducing emulsion was injected subcutaneously at the base of the rat's tail, and on Day 8, 100 µL of the inducing emulsion was injected subcutaneously again at a different site on the base of the rat's tail. All the rats were confirmed to successfully develop CIA by Day 17 (hereinafter referred to as "CIA rat").

### Example 1: Effect of amino acid-chelated zinc on dendritic cell maturation

The secretion level of IL12P70 in immature DCs is very low, whereas mature DCs secret a large amount of IL12P70, allowing it an indicator of DC maturation. In this example, the expression levels of the surface markers (CD11c, CD40, CD83, CD86) on the BMDC were analyzed through flow cytometry, and the secretion level of IL12p70 in the BMDC medium was measured with ELISA, to observe the effects of amino acid-chelated zinc on dendritic cell maturation.

### (1-1) Cell treatment

The BMDC obtained from Preparation Example B were seeded to a 24-well plate at a concentration of 1×10⁵ cells/well, and the cells in each group were cultured with 500 µL of the following culture medium at 37°C and 5% CO₂ for 48 hours:
(1) "Immature DC" group: DC medium;
(2) "Mature DC" group: DC medium containing 1000 U/mL of IFN-γ, 2.5 µg/mL of R848, 20 µg/mL of poly I:C and 10 ng/mL of LPS;
(3) "Zinc sulfate (low concentration)" group: DC medium containing zinc sulfate at a zinc concentration of 3.27 mg/mL;
(4) "Zinc sulfate (medium concentration)" group: DC medium containing zinc sulfate at a zinc concentration of 6.54 mg/mL;
(5) "Zinc sulfate (high concentration)" group: DC medium containing zinc sulfate at a zinc concentration of 13.08 mg/mL;
(6) "CMC-Zn (low concentration)" group: the CMC-Zn at a zinc concentration of 3.27 mg/mL provided by Preparation Example A;
(7) "CMC-Zn (medium concentration)" group: the CMC-Zn at a zinc concentration of 6.54 mg/mL provided by Preparation Example A;
(8) "CMC-Zn (high concentration)" group: the CMC-Zn at a zinc concentration of 13.08 mg/mL provided by Preparation Example A;
(9) "Modified CMC-Zn (low concentration)" group: the modified CMC-Zn at a zinc concentration of 3.27 mg/mL provided by Preparation Example A;
(10) "Modified CMC-Zn (medium concentration)" group: the modified CMC-Zn at a zinc concentration of 6.54 mg/mL provided by Preparation Example A;
(11) "Modified CMC-Zn (high concentration)" group: the modified CMC-Zn at a zinc concentration of 13.08 mg/mL provided by Preparation Example A;

After culturing for 48 hours, the cells and their medium in each group were collected to conduct subsequent analysis, respectively.

### (1-2) Flow cytometry analysis

The cells obtained from (1-1) were counted and the cell concentration of the cell suspension was adjusted with PBS to 1×10⁵ cells to 5×10⁵ cells per 100 µL. 100 µL of the cell suspension was evenly mixed with 20 µL of FcR Blocking Reagent, and the mixture thus obtained was placed at 4°C for 10 minutes. Then, the antibodies of CD11c, CD40, CD80, CD83, CD86, MHC II and corresponding isotype control were added into the tubes containing the mixture, respectively. After vortexing for 1 second, the tubes were placed at room temperature in the dark for 15 minutes. Then, 300 µL of PBS was added into each tube, and after vortexing for 1 second, the tubes were centrifuged at 200 xg for 5 minutes. The supernatants after centrifuging were removed and the cells were re-suspended with 300 µL of PBS. Finally, the cell suspensions thus obtained were analyzed by flow cytometry, wherein all the cell suspension of each group were kept on ice in the dark until the analysis was conducted.

As shown in Table 1 and Figs. 2A to 2K, the induced mature BMDCs and/or the BMDCs treated with zinc sulfate, CMC-Zn or modified CMC-Zn were analyzed by flow cytometry to determine the expression levels of the dendritic cell maturation markers (CD40, CD80, CD83, CD86 and MHC II). The analysis results showed that the expression levels of the markers on the induced mature BMDC were all increased, indicating that the study platform was reliable. All the groups of mature DC and zinc sulfate, CMC-Zn and modified CMC-Zn demonstrated the ability to inhibit dendritic cell maturation, proportional to the zinc concentration. CMC-Zn and modified CMC-Zn at a low concentration were effective in inhibiting DC maturation, with their expression levels of the markers even lower than those of the "Immature DC" group. As compared to the groups of zinc sulfate, the expression levels of CD80 and CD86 in the groups of CMC-Zn and modified CMC-Zn at both low and medium concentration were significantly decreased. The experimental results indicated that the abilities of CMC-Zn and modified CMC-Zn to inhibit dendritic cell maturation were better than that of zinc sulfate. The aforesaid experimental results demonstrated that the amino acid-chelated zinc was indeed effective in inhibiting DC maturation, and thus could be used for immune regulation in the treatment of an allergic disease, the treatment of an autoimmune disease, the prevention of an organ transplant rejection and the like, and for the promotion of the generation of tolerogenic DCs that could serve as an immunosuppressant.

**Table 1**

| Groups | CD11c | CD40 | CD80 | CD83 | CD86 | MHC II |
|---|---|---|---|---|---|---|
| Immature DC | 91 | 29.8 | 47.7 | 17.6 | 47 | 29. |
| Mature DC | 77.6 | 84.2 | 66.8 | 33.6 | 67.2 | 84.3 |
| Zinc sulfate (low concentration) | 87.2 | 16.1 | 46.7 | 12.9 | 49.2 | 16.8 |
| Zinc sulfate (medium concentration) | 85.4 | 11.2 | 37.2 | 10.4 | 41.3 | 11.9 |
| Zinc sulfate (high concentration) | 85.6 | 8.5 | 26.8 | 6.68 | 26 | 8.08 |
| CMC-Zn (low concentration) | 88.3 | 25 | 39 | 16.1 | 37.9 | 25 |
| CMC-Zn (medium concentration) | 87.8 | 13.1 | 23.7 | 10.3 | 23.1 | 14 |
| CMC-Zn (high concentration) | 74.1 | 6.24 | 26.5 | 4.38 | 25.4 | 6.41 |
| Modified CMC-Zn (low concentration) | 86.6 | 14.7 | 30.7 | 10.4 | 29.1 | 14.9 |
| Modified CMC-Zn (medium concentration) | 83.5 | 7.49 | 24.8 | 4.98 | 25.1 | 8.46 |
| Modified CMC-Zn (high concentration) | 57.9 | 5.1 | 27.5 | 1.3 | 27 | 4.91 |

### (1-3) ELISA analysis

The media obtained from each group of (1-1) were placed into centrifuge tubes and centrifuged (400 xg) at 4°C for 10 minutes to collect the supernatants. The IL12p70 standard was subjected to a serial dilution to provide a standard curve. The standards at various concentrations and the supernatants obtained from each group were added into a 96-well plate pre-coating with IL12p70 antibodies (100 µL per well, in duplicate). The 96-well plate was gently tapped to ensure the liquid in each well was evenly mixed, and the 96-well plate was placed in a 37°C incubator for 1.5 hours. Then, 350 µL of 1X wash buffer was added into each well of the plate, and after soaking for 30 seconds, the liquid in each well was removed, and the washing step was repeated four more times. After washing, 100 µL of 1X antibody conjugates was added into each well, and the 96-well plate was gently tapped to ensure the liquid in each well was evenly mixed. The 96-well plate was placed in the 37°C incubator for 1 hour. Again, the washing step was repeated five times by using the 1X wash buffer. Then, 100 µL of 1X HPR-Streptavidin solution was added into each well, and after gently tapping, the 96-well plate was placed in the 37°C incubator in the dark. After 30 minutes, the washing step was repeated five times again. Thereafter, 100 µL of TMB reagent was added into each well, and after gently tapping, the 96-well plate was placed in the 37°C incubator in the dark for 15 minutes. The 96-well plate was removed from the incubator, and 100 µL of Stop Solution was added into each well. The absorbance value at 450 nanometer (nm) (OD450 nm) in each well was detected within 3 minutes. Then, based on the absorbance values of standards at various concentrations, a standard curve was plotted to obtain the IL12p70 amounts corresponding to the absorbance values of each group, as shown in Table 2.

As shown in Table 2, except for the cell culture medium of the "Mature DC" group, IL12P70 was not detected in the media obtained from the "Immature DC" group and the groups of zinc sulfate, CMC-Zn and modified CMC-Zn. These results once again demonstrated that the amino acid-chelated zinc was effective in inhibiting DC maturation, and thus could be used for immune regulation in the treatment of an allergic disease, the treatment of an autoimmune disease, the prevention of an organ transplant rejection and the like, and for the promotion of the generation of tolerogenic DCs that could serve as an immunosuppressant.

**Table 2**

| Groups | IL12P70 amount (pg/mL) |
|---|---|
| Immature DC | ND |
| Mature DC | 113.06 |
| Zinc sulfate (low concentration) | ND |
| Zinc sulfate (medium concentration) | ND |
| Zinc sulfate (high concentration) | ND |
| Modified CMC-Zn (low concentration) | ND |
| Modified CMC-Zn (medium concentration) | ND |
| Modified CMC-Zn (high concentration) | ND |
| Modified CMC-Zn (low concentration) | ND |
| Modified CMC-Zn (medium concentration) | ND |
| Modified CMC-Zn (high concentration) | ND |

| | |
|---|---|
| *ND: not detected | |

### Example 2: Effect of amino acid-chelated zinc on autoimmune disease

Rheumatoid arthritis is an autoimmune disease in which the patient's immune system is abnormally activated to attack its own cells and tissues, thereby causing a long-term chronic inflammation in the body. In this example, the therapeutic effects of amino acid-chelated zinc on autoimmune diseases such as rheumatoid arthritis were observed by a CIA animal model.

As described in [Preparation Example] C, it was confirmed on Day 17 that all CIA rats were successfully induced. Therefore, the CIA rats were randomly divided into four groups from Day 17 to administer respectively the following solutions until the rats were sacrificed on Day 24, wherein the healthy rats without induced arthritis were served as the control group:
(1) Control group (five healthy rats): 2 mL of PBS;
(2) CIA group (five CIA rats): 2 mL of PBS;
(3) MTX group (three CIA rats): 1 mg of MTX per kilogram of rat, dissolved in 2 mL of PBS;
(4) CMC-Zn group (five CIA rats): CMC-Zn at a zinc concentration of 0.075 mg, dissolved in 2 mL of PBS; and
(5) Modified CMC-Zn group (five CIA rats): modified CMC-Zn at a zinc concentration of 0.075 mg, dissolved in 2 mL of PBS.

The rat's paws of each group were observed and their volumes were measured at the beginning of the experiment and on Day 3, 7, 10, 14, 17, 21 and 24, wherein the arthritis severity of rats was evaluated by using the qualitative scoring system standard of Table 3, while the paw volume was determined by the following approach in accordance with the buoyancy principle of Archimedes' law: adding an appropriate amount of water into a beaker; placing the beaker on a scale and resetting to zero; placing the rat's paw into the water and immersing it to the target depth; and recoding the reading once it had stabilized.

**Table 3**

| Degrees of severity | Score |
|---|---|
| Normal | 0 |
| The ankle or wrist has mild but obvious redness and swelling; or the individual finger has obvious redness and swelling (regardless of the number of fingers). | 1 |
| The ankle or wrist has moderate redness and swelling. | 2 |
| The whole paw (including the fingers) has severe redness and swelling. | 3 |
| Widespread inflammation of the limbs, involving multiple joints | 4 |

As shown in Fig. 3, except for the paws of the healthy rats in the control group, all the weight percentages of the CIA rats' paws were markedly increased on Day 17, indicating that the CIA rats all successfully developed arthritis. As compared to the rats of the CIA group, the weight percentages of the rats' paws in the MTX group, CMC-Zn group and Modified CMC-Zn group were all gradually decreased after being administered MTX, CMC-Zn or modified CMC-Zn, respectively. This indicated that the arthritis symptoms of the CIA rats administered with MTX, CMC-Zn or modified CMC-Zn were gradually improved, wherein the effect of modified CMC-Zn was even comparable to that of MTX. Fig. 4 showed that on Day 24, as compared to the rats of the control group, the weight percentage of the CIA rats' paws were all significantly increased, and as compared to the rats of the untreated CIA rats, the weight percentages of the rats' paws in the MTX group, CMC-Zn group and Modified CMC-Zn group were all significantly decreased. The aforesaid results showed that the administration of the amino acid-chelated zinc to a subject was indeed effective in alleviating the symptoms of rheumatoid arthritis, and thus, the amino acid-chelated zinc could be used for treating autoimmune disease such as rheumatoid arthritis.

Table 4 showed the summed average scores of arthritis severity of both paws at the start of the experiment (Days 0), and on Day 3, 7, 10, 14, 17, 21 and 24 in each group of rats, rated according to the criteria listed in Table 3. As compared to the CIA group, the rats in the MTX group, CMC-Zn group and Modified CMC-Zn group (i.e., after administering MTX, CMC-Zn or modified CMC-Zn) showed slower progression, or even reduction, in arthritis severity after Day 17. Especially, the score of arthritis severity of rats in the Modified CMC-Zn group was even lower than that of the MTX group.

**Table 4**

| | Day0 | Day3 | Day7 | Day10 | Day14 | Day 17 | Day21 | Day24 |
|---|---|---|---|---|---|---|---|---|
| Control group | 0 | 0 | 0 | 0 | 0 | 0 | 0 | 0 |
| CIA group | 0 | 0 | 0 | 0 | 0.8 | 3.6 | 6.0 | 6.6 |
| MTX group | 0 | 0 | 0 | 0 | 0.3 | 3.7 | 3.7 | 3 |
| CMC-Zn group | 0 | 0 | 0 | 0 | 0.4 | 3.8 | 4.4 | 4.6 |
| Modified CMC-Zn group | 0 | 0 | 0 | 0 | 1 | 4.2 | 2 | 1.8 |

As can be seen from the aforementioned results, the amino acid-chelated zinc can not only effectively inhibit DC maturation, but can also effectively alleviate the related symptoms in subjects with rheumatoid arthritis. Therefore, the amino acid-chelated zinc can be used for immune regulation in the treatment of an allergic disease, the treatment of an autoimmune disease, the prevention of an organ transplant rejection and the like, and for the promotion of the generation of tolerogenic DCs that could serve as an immunosuppressant.

The above embodiments are merely to explain the principles and effects of the present invention and to describe its technical features, and are not intended to limit the scope of the present invention. Any modifications or arrangements that a person skilled in this field can easily achieve without deviating from the principle of the present invention fall within the scope claimed by the present invention. Therefore, the scope of protection of the present invention is defined by the claims appended hereto.

## Claims

1. Use of an amino acid-chelated zinc in the manufacture of a composition, **characterized in that** the composition is for use in regulating immunity.

2. The use of claim 1 **characterized in that** the composition is an aqueous composition with a pH less than 7.

3. The use of claim 1 **characterized in that** the amino acid-chelated zinc has a molar ratio of zinc and amino acid ranging from 1: 1 to 1: 10.

4. The use of claim 1 **characterized in that** the amino acid-chelated zinc is glycine-chelated zinc, alanine-chelated zinc, valine-chelated zinc, leucine-chelated zinc, and/or isoleucine-chelated zinc.

5. The use of any one of claims 1 to 4 **characterized in that** the composition is a pharmaceutical composition, and the pharmaceutical composition is for use in treating an allergic disease, treating an autoimmune disease, and/or preventing an organ transplant rejection.

6. Use of an amino acid-chelated zinc in the manufacture of a composition **characterized in that** the composition is for use in inhibiting dendritic cell maturation.

7. The use of claim 6 **characterized in that** the composition is an aqueous composition with a pH less than 7.

8. The use of claim 6 **characterized in that** the amino acid-chelated zinc has a molar ratio of zinc and amino acid ranging from 1: 1 to 1: 10.

9. The use of claim 6 **characterized in that** the amino acid-chelated zinc is glycine-chelated zinc, alanine-chelated zinc, valine-chelated zinc, leucine-chelated zinc, and/or isoleucine-chelated zinc.

10. A combination **characterized in that** the composition comprises:
(1) an amino acid-chelated zinc; and
(2) a dendritic cell medium.

11. The combination of claim 10 **characterized in that** the amino acid-chelated zinc has a molar ratio of zinc and amino acid ranging from 1: 1 to 1: 10.

12. The combination of claim 10 **characterized in that** the amino acid-chelated zinc is glycine-chelated zinc, alanine-chelated zinc, valine-chelated zinc, leucine-chelated zinc, and/or isoleucine-chelated zinc.

13. The combination of claim 10 **characterized in that** the composition further comprises a dendritic cell.

14. The combination of any one of claims 10 to 13 **characterized in that** based on the amount of the dendritic cell medium, the combination comprises from 1 mg to 25 mg of the amino acid-chelated zinc per mL of the dendritic cell medium.

15. A composition for use in regulating immunity **characterized in that** the composition comprises an effective amount of an amino acid-chelated zinc.

16. The composition of claim 15 **characterized in that** the composition is an aqueous composition with a pH less than 7.

17. The composition of claim 15 **characterized in that** the amino acid-chelated zinc has a molar ratio of zinc and amino acid ranging from 1: 1 to 1: 10.

18. The composition of claim 15 **characterized in that** the amino acid-chelated zinc is glycine-chelated zinc, alanine-chelated zinc, valine-chelated zinc, leucine-chelated zinc, and/or isoleucine-chelated zinc.

19. The composition of any one of claims 15 to 18 **characterized in that** the composition is a pharmaceutical composition and the pharmaceutical composition is for treating an allergic disease, treating an autoimmune disease and/or preventing an organ transplant rejection.

20. A composition for use in inhibiting dendritic cell maturation **characterized in that** the composition comprises an effective amount of an amino acid-chelated zinc.

21. The composition of claim 20 **characterized in that** the composition is an aqueous composition with a pH less than 7.

22. The composition of claim 20 **characterized in that** the amino acid-chelated zinc has a molar ratio of zinc and amino acid ranging from 1: 1 to 1: 10.

23. The composition of claim 20 **characterized in that** the amino acid-chelated zinc is glycine-chelated zinc, alanine-chelated zinc, valine-chelated zinc, leucine-chelated zinc, and/or isoleucine-chelated zinc.
